(19)

Europäisches Patentamt
European Patent Office
Office européen des brevets

(11) **EP 3 862 017 A1**

(12) **EUROPEAN PATENT APPLICATION**
published in accordance with Art. 153(4) EPC

(43) Date of publication:
**11.08.2021 Bulletin 2021/32**

(21) Application number: **19869158.6**

(22) Date of filing: **04.10.2019**

(51) Int Cl.:
**A61K 38/16** (2006.01)   **A61P 1/04** (2006.01)
**A61P 29/00** (2006.01)   **A61P 37/02** (2006.01)
**A61P 43/00** (2006.01)

(86) International application number:
**PCT/JP2019/039232**

(87) International publication number:
**WO 2020/071520 (09.04.2020 Gazette 2020/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.10.2018 JP 2018190090**

(71) Applicants:
• **Stemrim Inc.**
  **Ibaraki-shi, Osaka 567-0085 (JP)**
• **Osaka University**
  **Suita-shi, Osaka 565-0871 (JP)**

(72) Inventors:
• **TAMAI, Katsuto**
  **Suita-shi, Osaka 565-0871 (JP)**
• **SHIMBO, Takashi**
  **Suita-shi, Osaka 565-0871 (JP)**
• **YAMAZAKI, Takehiko**
  **Ibaraki-shi, Osaka 567-0085 (JP)**
• **YOKOTA, Koichi**
  **Ibaraki-shi, Osaka 567-0085 (JP)**

(74) Representative: **Zacco GmbH**
**Bayerstrasse 83**
**80335 München (DE)**

(54) **PEPTIDE POSSESSING MESENCHYMAL-STEM-CELL MOBILIZING ACTIVITY**

(57)    The present inventors designed an artificial sequence peptide based on the results of original research conducted so far, and found that the peptide exhibits an activity of mobilizing mesenchymal stem cells into peripheral blood. The present inventors also found that the artificial sequence peptide has a therapeutic effect on inflammatory bowel disease, atopic dermatitis, and cerebral infarction. Based on these findings, a new regenerative medicine technology that can overcome the problems of cell transplantation therapy is provided.

FIG. 1

SEQUENCE LISTING

```
<110>   StemRIM Inc.
        Osaka University

<120>   Peptides having an activity of mobilizing mesenchymal stem cells

<130>   G6-A1805P

<150>   JP 2018-190090
<151>   2018-10-05

<160>   2

<170>   PatentIn version 3.5

<210>   1
<211>   30
<212>   PRT
<213>   Artificial Sequence

<220>
<223>   An artificially synthesized peptide sequence

<400>   1

Ile Met Trp Leu Cys Gln Tyr Phe Phe Gln Cys His Lys Cys Ala Val
1               5                   10                  15


Lys Lys Lys Thr Trp Lys Glu Lys Lys Asp Arg Arg His Pro
            20                  25                  30


<210>   2
<211>   90
<212>   DNA
<213>   Artificial Sequence

<220>
<223>   An artificially synthesized nucleotide sequence

<400>   2
atcatgtggc tgtgccagta cttcttccag tgccacaagt gcgccgtgaa gaagaagacc         60

tggaaggaga agaaggacag gaggcacccc                                          90
```

**Description**

[Technical Field]

The present application relates to peptides having an activity of mobilizing mesenchymal stem cells, compositions for mobilizing mesenchymal stem cells, and agents for treating diseases based on the mobilization of mesenchymal stem cells.

[Background Art]

Mesenchymal stem cells contained in bone marrow fluid and the like have the ability to differentiate into various tissues (pluripotency) such as bone, cartilage, fat, muscle, nerve, and epithelium. In recent years, attempts have been widely made to perform regenerative medicine (cell transplantation therapy) using bone marrow-derived mesenchymal stem cells proliferated by culture. However, collection of bone marrow blood containing mesenchymal stem cells is done with an invasive technique which inserts a thick needle into the iliac bone repeatedly, thereby placing a large burden on the donor. In addition, mesenchymal stem cells gradually lose their proliferative ability and pluripotency when subcultured continuously in vitro. Furthermore, culturing mesenchymal stem cells based on high quality control that guarantees the safety of in vivo transplantation requires special culture equipment such as CPC (cell processing center), so the current situation is that it can be carried out only at a limited number of universities and companies.

[Citation List]

[Non-Patent Literature]

[NPL 1] PNAS 2011 Apr 19; 108 (16): 6609-14

[Patent Literature]

[PTL 1] WO2008/053892
[PTL 2] WO2009/133939
[PTL 3] WO2012/147470

[Summary of Invention]

[Technical Problem]

An objective of the present application is to develop a new regenerative medicine technology that can overcome the problems of cell transplantation therapy.

[Solution to Problem]

The present inventors designed an artificial sequence peptide based on the results of original research conducted so far, and discovered that the peptide exhibits an activity of mobilizing mesenchymal stem cells into peripheral blood. It was also discovered that the artificial sequence peptide has therapeutic effects on inflammatory bowel disease, atopic dermatitis, and cerebral infarction. Based on these findings, the present application provides a new regenerative medicine technology that can overcome the problems of cell transplantation therapy.
Specifically, the present application provides the following:

[1] A composition for use in mobilizing mesenchymal stem cells to peripheral blood, which comprises a peptide selected from the following:

(a) a peptide comprising the amino acid sequence of SEQ ID NO: 1;
(b) a peptide comprising an amino acid sequence resulting from substitution, deletion, insertion, or addition of one or several amino acids in the amino acid sequence of SEQ ID NO: 1; and
(c) a peptide comprising an amino acid sequence with a sequence identity of about 90% or higher with the amino acid sequence of SEQ ID NO: 1.

[2] A composition for use in treatment of a disease or pathological condition in a subject by mobilizing mesenchymal stem cells to peripheral blood, which comprises a peptide selected from the following:

> (a) a peptide comprising the amino acid sequence of SEQ ID NO: 1;
> (b) a peptide comprising an amino acid sequence resulting from substitution, deletion, insertion, or addition of one or several amino acids in the amino acid sequence of SEQ ID NO: 1; and
> (c) a peptide comprising an amino acid sequence with a sequence identity of about 90% or higher with the amino acid sequence of SEQ ID NO: 1.

[3] The composition of [2], wherein the treatment of a disease or pathological condition is selected from inflammation-suppressing therapy, immunomodulatory therapy, tissue regeneration-inducing therapy, and tissue fibrosis-suppressing therapy.

[4] The composition of [2], wherein the disease or pathological condition is selected from an inflammatory disease, an autoimmune disease, a disease accompanied by tissue damage, ischemia, or necrosis, and a fibrotic disease.

[5] The composition of [2], wherein the disease or pathological condition is inflammatory bowel disease.

[6] The composition of [2], wherein the disease or pathological condition is ulcerative colitis.

[7] The composition of [2], wherein the disease or pathological condition is atopic dermatitis.

[8] The composition of [2], wherein the disease or pathological condition is cerebral infarction.

[9] A composition for use in treatment of a disease selected from inflammatory bowel disease, atopic dermatitis, and cerebral infarction, which comprises a peptide selected from the following:

> (a) a peptide comprising the amino acid sequence of SEQ ID NO: 1;
> (b) a peptide comprising an amino acid sequence resulting from substitution, deletion, insertion, or addition of one or several amino acids in the amino acid sequence of SEQ ID NO: 1; and
> (c) a peptide comprising an amino acid sequence with a sequence identity of about 90% or higher with the amino acid sequence of SEQ ID NO: 1.

[10] A peptide selected from the following:

> (a) a peptide comprising the amino acid sequence of SEQ ID NO: 1;
> (b) a peptide comprising an amino acid sequence resulting from substitution, deletion, insertion, or addition of one or several amino acids in the amino acid sequence of SEQ ID NO: 1; and
> (c) a peptide comprising an amino acid sequence with a sequence identity of about 90% or higher with the amino acid sequence of SEQ ID NO: 1.

[A1] A method for mobilizing mesenchymal stem cells to peripheral blood, comprising administering to a subject an effective amount of a peptide selected from the following:

> (a) a peptide comprising the amino acid sequence of SEQ ID NO: 1;
> (b) a peptide comprising an amino acid sequence resulting from substitution, deletion, insertion, or addition of one or several amino acids in the amino acid sequence of SEQ ID NO: 1; and
> (c) a peptide comprising an amino acid sequence with a sequence identity of about 90% or higher with the amino acid sequence of SEQ ID NO: 1.

[A2] A method for treating a disease or pathological condition in a subject by mobilizing mesenchymal stem cells to peripheral blood, which comprises administering to the subject an effective amount of a peptide selected from the following:

> (a) a peptide comprising the amino acid sequence of SEQ ID NO: 1;
> (b) a peptide comprising an amino acid sequence resulting from substitution, deletion, insertion, or addition of one or several amino acids in the amino acid sequence of SEQ ID NO: 1; and
> (c) a peptide comprising an amino acid sequence with a sequence identity of about 90% or higher with the amino acid sequence of SEQ ID NO: 1.

[A3] The method of [A2], wherein the treatment of a disease or pathological condition is selected from inflammation-suppressing therapy, immunomodulatory therapy, tissue regeneration-inducing therapy, and tissue fibrosis-suppressing therapy.

[A4] The method of [A2], wherein the disease or pathological condition is selected from an inflammatory disease, an autoimmune disease, a disease accompanied by tissue damage, ischemia, or necrosis, and a fibrotic disease.

[A5] The method of [A2], wherein the disease or pathological condition is inflammatory bowel disease.

[A6] The method of [A2], wherein the disease or pathological condition is ulcerative colitis.

[A7] The method of [A2], wherein the disease or pathological condition is atopic dermatitis.

[A8] The method of [A2], wherein the disease or pathological condition is cerebral infarction.

[A9] A method for treating a disease selected from inflammatory bowel disease, atopic dermatitis, and cerebral infarction in a subject, which comprises administering to the subject an effective amount of a peptide selected from the following:

(a) a peptide comprising the amino acid sequence of SEQ ID NO: 1;
(b) a peptide comprising an amino acid sequence resulting from substitution, deletion, insertion, or addition of one or several amino acids in the amino acid sequence of SEQ ID NO: 1; and
(c) a peptide comprising an amino acid sequence with a sequence identity of about 90% or higher with the amino acid sequence of SEQ ID NO: 1.

[B1] A peptide for use in mobilizing mesenchymal stem cells to peripheral blood, which is selected from the following:

(a) a peptide comprising the amino acid sequence of SEQ ID NO: 1;
(b) a peptide comprising an amino acid sequence resulting from substitution, deletion, insertion, or addition of one or several amino acids in the amino acid sequence of SEQ ID NO: 1; and
(c) a peptide comprising an amino acid sequence with a sequence identity of about 90% or higher with the amino acid sequence of SEQ ID NO: 1.

[B2] A peptide for use in treatment of a disease or pathological condition in a subject by mobilizing mesenchymal stem cells to peripheral blood, which is selected from the following:

(a) a peptide comprising the amino acid sequence of SEQ ID NO: 1;
(b) a peptide comprising an amino acid sequence resulting from substitution, deletion, insertion, or addition of one or several amino acids in the amino acid sequence of SEQ ID NO: 1; and
(c) a peptide comprising an amino acid sequence with a sequence identity of about 90% or higher with the amino acid sequence of SEQ ID NO: 1.

[B3] The peptide of [B2], wherein the treatment of a disease or pathological condition is selected from inflammation-suppressing therapy, immunomodulatory therapy, tissue regeneration-inducing therapy, and tissue fibrosis-suppressing therapy.

[B4] The peptide of [B2], wherein the disease or pathological condition is selected from an inflammatory disease, an autoimmune disease, a disease accompanied by tissue damage, ischemia, or necrosis, and a fibrotic disease.

[B5] The peptide of [B2], wherein the disease or pathological condition is inflammatory bowel disease.

[B6] The peptide of [B2], wherein the disease or pathological condition is ulcerative colitis.

[B7] The peptide of [B2], wherein the disease or pathological condition is atopic dermatitis.

[B8] The peptide of [B2], wherein the disease or pathological condition is cerebral infarction.

[B9] A peptide for use in treatment of a disease selected from inflammatory bowel disease, atopic dermatitis, and cerebral infarction, which is selected from the following:

(a) a peptide comprising the amino acid sequence of SEQ ID NO: 1;
(b) a peptide comprising an amino acid sequence resulting from substitution, deletion, insertion, or addition of one or several amino acids in the amino acid sequence of SEQ ID NO: 1; and
(c) a peptide comprising an amino acid sequence with a sequence identity of about 90% or higher with the amino acid sequence of SEQ ID NO: 1.

[C1] Use of a peptide selected from the following in manufacture of a medicament or reagent for mobilizing mesenchymal stem cells to peripheral blood:

(a) a peptide comprising the amino acid sequence of SEQ ID NO: 1;
(b) a peptide comprising an amino acid sequence resulting from substitution, deletion, insertion, or addition of one or several amino acids in the amino acid sequence of SEQ ID NO: 1; and
(c) a peptide comprising an amino acid sequence with a sequence identity of about 90% or higher with the amino acid sequence of SEQ ID NO: 1.

[C2] Use of a peptide selected from the following in manufacture of a medicament for treating a disease or pathological condition in a subject by mobilizing mesenchymal stem cells to peripheral blood:

(a) a peptide comprising the amino acid sequence of SEQ ID NO: 1;
(b) a peptide comprising an amino acid sequence resulting from substitution, deletion, insertion, or addition of one or several amino acids in the amino acid sequence of SEQ ID NO: 1; and
(c) a peptide comprising an amino acid sequence with a sequence identity of about 90% or higher with the amino acid sequence of SEQ ID NO: 1.

[C3] The use of [C2], wherein the treatment of a disease or pathological condition is selected from inflammation-suppressing therapy, immunomodulatory therapy, tissue regeneration-inducing therapy, and tissue fibrosis-suppressing therapy.

[C4] The use of [C2], wherein the disease or pathological condition is selected from an inflammatory disease, an autoimmune disease, a disease accompanied by tissue damage, ischemia, or necrosis, and a fibrotic disease.

[C5] The use of [C2], wherein the disease or pathological condition is inflammatory bowel disease.

[C6] The use of [C2], wherein the disease or pathological condition is ulcerative colitis.

[C7] The use of [C2], wherein the disease or pathological condition is atopic dermatitis.

[C8] The use of [C2], wherein the disease or pathological condition is cerebral infarction.

[C9] Use of a peptide selected from the following in manufacture of a medicament for treating a disease selected from inflammatory bowel disease, atopic dermatitis, and cerebral infarction:

(a) a peptide comprising the amino acid sequence of SEQ ID NO: 1;
(b) a peptide comprising an amino acid sequence resulting from substitution, deletion, insertion, or addition of one or several amino acids in the amino acid sequence of SEQ ID NO: 1; and
(c) a peptide comprising an amino acid sequence with a sequence identity of about 90% or higher with the amino acid sequence of SEQ ID NO: 1.

[Brief Description of Drawings]

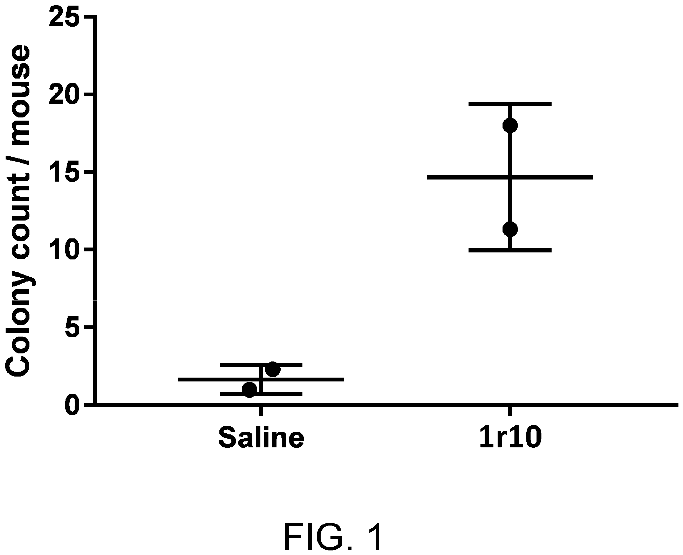

[Figure 1] Fig. 1 is a plot of the number of colonies obtained by culturing peripheral blood collected 14 hours after administration of physiological saline solution or the peptide. In the graph, "saline" indicates the control group, and "1r10" indicates the peptide 1r10 administration group. The number of colonies was shown as the value per peripheral blood volume (about 800 $\mu$L) collected from one mouse. The long horizontal bar represents the mean value, and the short bar represents the standard deviation.

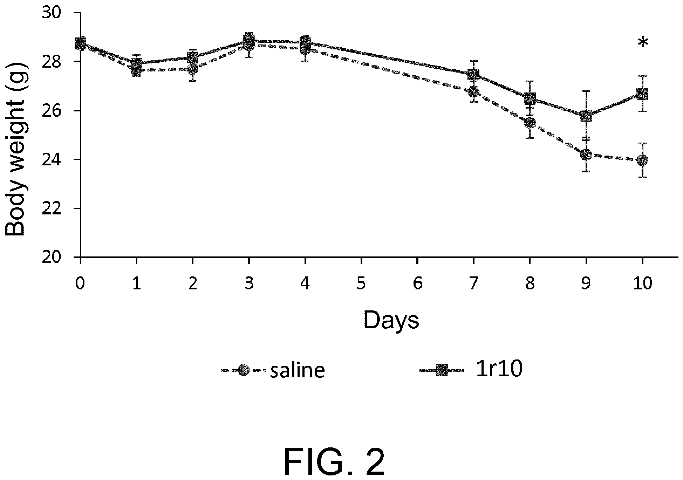

[Figure 2] Fig. 2 is a graph showing changes in mouse body weight (mean $\pm$ standard error (SEM); * p <0.05 (Student's t test)). In the graph, "saline" indicates the control group, and "1r10" indicates the peptide 1r10 administration group. The horizontal axis shows the number of days after the start of drinking an aqueous solution of dextran sulfate sodium (DSS).

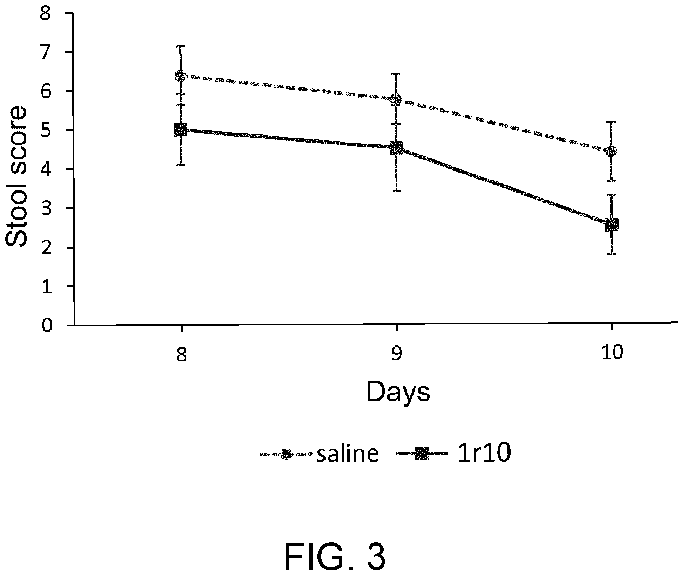

[Figure 3] Fig. 3 is a graph showing stool scores of mice (mean $\pm$ standard error (SEM)). In the graph, "saline" indicates the control group, and "1r10" indicates the peptide 1r10 administration group. The horizontal axis shows the number of days after the start of drinking an aqueous solution of dextran sulfate sodium (DSS).

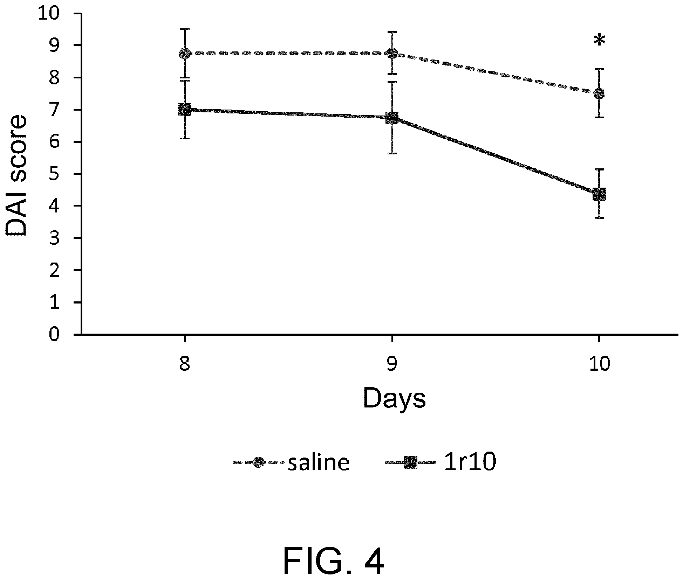

[Figure 4] Fig. 4 is a graph showing DAI scores of mice (mean $\pm$ standard error (SEM); * p <0.05 (Mann Whitney U test)). In the graph, "saline" indicates the control group, and "1r10" indicates the peptide 1r10 administration group. The horizontal axis shows the number of days after the start of drinking an aqueous solution of dextran sulfate sodium (DSS).

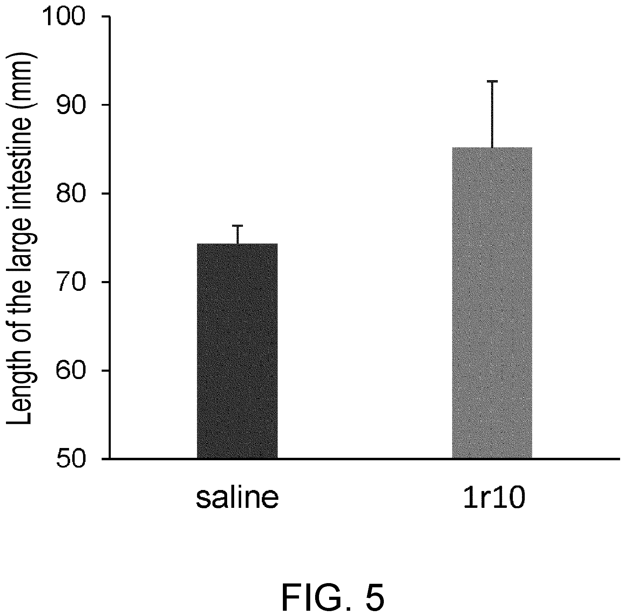

[Figure 5] Fig. 5 is a graph showing the length of the large intestine 10 days after the start of drinking an aqueous solution of dextran sulfate sodium (DSS) (mean $\pm$ standard error (SEM)). In the graph, "saline" indicates the control group, and "1r10" indicates the peptide 1r10 administration group.

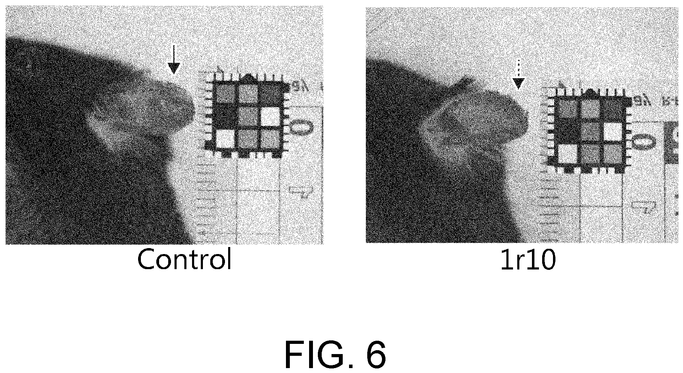

[Figure 6] Fig. 6 presents photographs showing the auricle of mice 4 days after the start of peptide administration. Arrows indicate the rim of the auricle where scaling is likely to be seen.

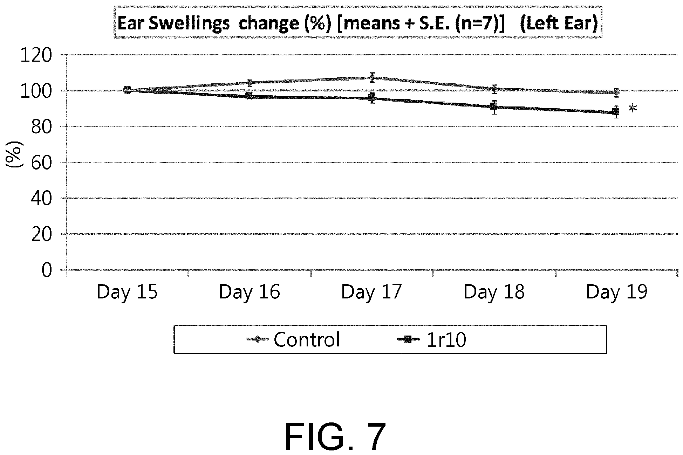

[Figure 7] Fig. 7 is a graph showing changes in auricular thickness during the peptide administration period (the vertical axis is a relative value with the start date of peptide administration as 100%). Each point shows the mean and standard error (* p <0.05; Repeated one way ANOVA test; post hoc Tukey-Kramer test).

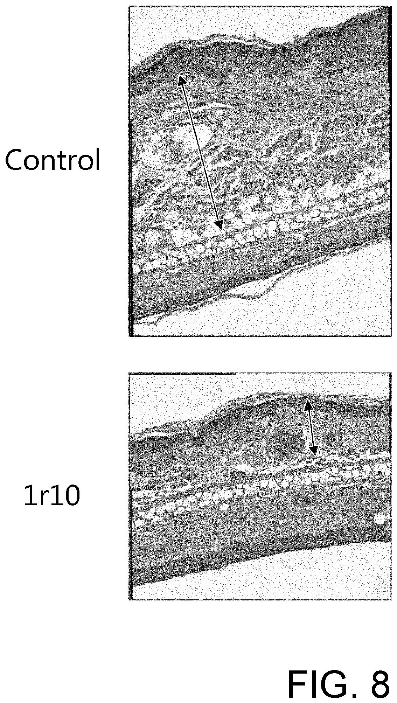

[Figure 8] Fig. 8 presents representative photographs showing H.E. stained images of skin sections of the auricle on the 4th day after the start of peptide administration. The arrows in the photographs show examples of the measured dermis thickness.

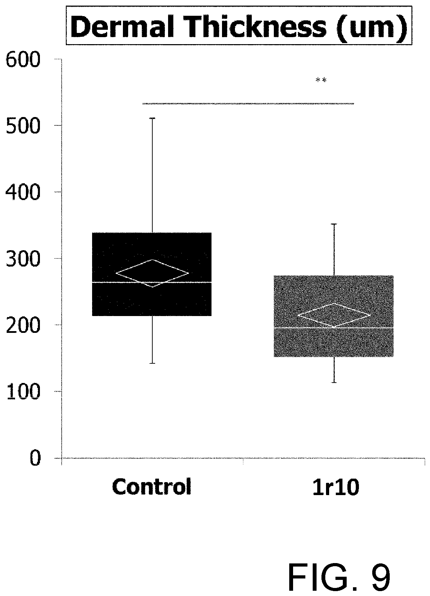

[Figure 9] Fig. 9 is a graph (Box-Whisker plot) showing the dermis thickness of each group on the 4th day after the start of peptide administration. The central Box and Whisker indicate the median, quartile, and maximum/minimum values. Diamond in the Box shows the average value. (** p <0.01 Mann Whitney U-test; 7 mice in each group x 9 measurements per mouse)

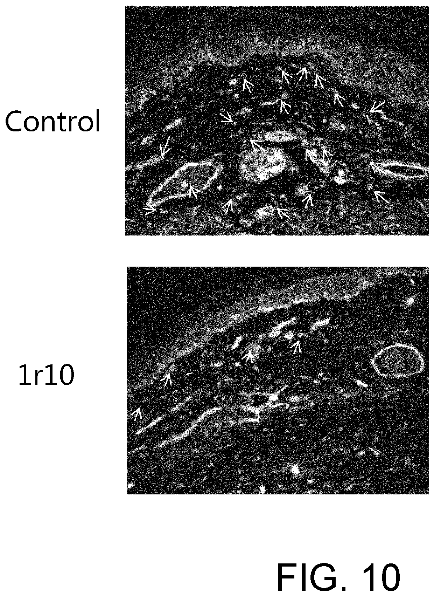

[Figure 10] Fig. 10 presents photographs showing the multiple fluorescence immunostaining images of skin sections of the auricle on the 4th day after the start of peptide administration (Laminin (green)-CD45 (red)-DAPI (blue)). Laminin was used to label structures in the tissue (blood vessels, basement membranes, nerves), and DAPI was used as a counterstain for the DNA of each cell.

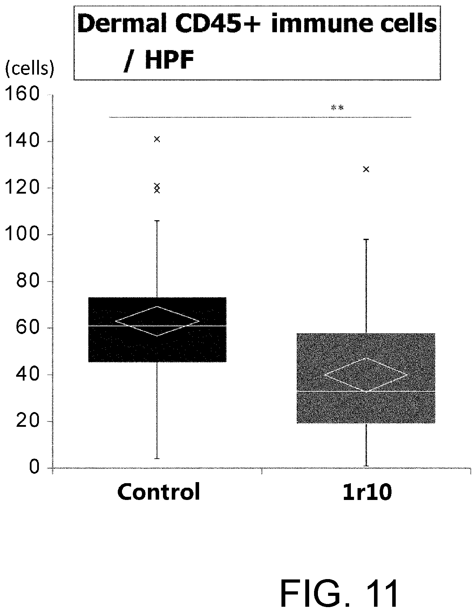

[Figure 11] Fig. 11 is a graph (Box-Whisker plot) showing the number of CD45-positive cells (CD45+ immune cells) per unit area of high power field (400x) in each group in the auricular skin sections 4 days after the start of peptide administration. The central Box and Whisker indicate the median, quartile, and maximum/minimum values. Diamond in the Box shows the average value. "x" represents the outlier. (** p <0.01 Mann Whitney U-test; 7 mice in each group x 9 measurements per mouse. Outliers appeared in 1 out of 7 individuals (about 14%) in both the control group and the peptide-administered group. Thus, for each group, the outlier was substituted with the average value of the other 6 individuals having no outliers.)

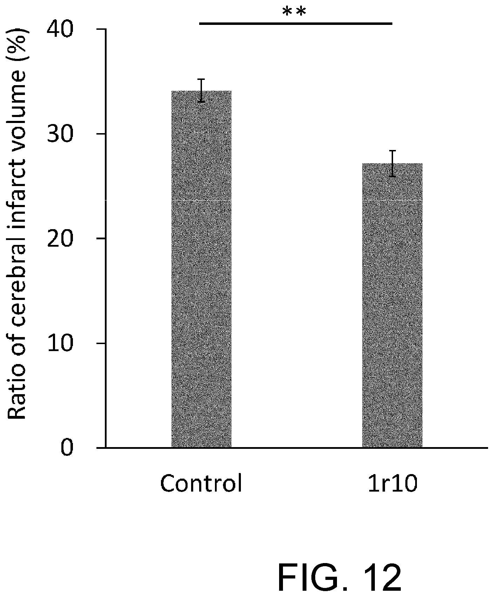

[Figure 12] Fig.12 is a graph showing the ratio of cerebral infarct volume 48 hours after right MCA occlusion (mean $\pm$ standard error). ** p <0.01, Mann-Whitney U test.

[Description of Embodiments]

The present inventors previously found a substance having an activity of activating stem cells in a living body or mobilizing them to an injured tissue via peripheral circulation, and believe that the substance is promising as a new type of medicine that can overcome the weaknesses of cell therapy. Specifically, the present inventors have found that High Mobility Group Box 1 (HMGB1), which is released from necrotic tissue, mobilizes cells that are positive for platelet-derived growth factor receptor $\alpha$ (PDGFR$\alpha$), which play a role in inducing tissue regeneration in vivo (believed to be mesenchymal stem cells (MSC)), to a necrotic tissue through peripheral circulation, thereby suppresses inflammation of the necrotic tissue and promotes tissue regeneration. Further, the present inventors have also found that fragment peptides of HMGB1 exhibit an activity of mobilizing mesenchymal stem cells into peripheral blood and a tissue regeneration-inducing activity. This time, as a result of designing an artificial sequence peptide having a specific amino acid sequence based on the results of original research conducted so far, the present inventors confirmed that the peptide demonstrates an activity of mobilizing mesenchymal stem cells into peripheral blood.

In addition, the present inventors discovered that the artificial sequence peptide shows therapeutic effects on inflammatory bowel diseases. Specifically, it was confirmed that the artificial sequence peptide improves the DAI (disease activity index) score, which is an index for evaluating disease activity, in a mouse model of inflammatory bowel disease.

In addition, the present inventors discovered that the artificial sequence peptide shows therapeutic effects on atopic dermatitis. Specifically, it was confirmed that the artificial sequence peptide suppresses scaling and edema, suppresses increases in dermal thickness, and suppresses infiltration of CD45-positive cells in a mouse model of atopic dermatitis.

In addition, the present inventors discovered that the artificial sequence peptide shows therapeutic effects on cerebral infarction. Specifically, it was confirmed that the artificial sequence peptide reduces the cerebral infarction lesion in a rat model of cerebral infarction.

It is well known to those skilled in the art that mesenchymal stem cells exert an anti-inflammatory action, immunomodulatory action, and anti-fibrotic action. In addition, since mesenchymal stem cells also have pluripotency that allows them to differentiate into various tissues, it is well known to those skilled in the art that they exert an action of promoting regeneration of injured tissues. Therefore, when an artificial sequence peptide having an activity of mobilizing mesenchymal stem cells into peripheral blood is administered to a subject, the mesenchymal stem cells are recruited into the peripheral blood, and the anti-inflammatory action, immunomodulatory action, anti-fibrotic action, tissue regeneration-promoting action (due to the differentiation and/or anti-inflammatory action of mesenchymal stem cells) of the mesenchymal stem cells are considered to bring about therapeutic effects on various diseases.

In the present application, the "artificial sequence peptide" refers to a peptide having an amino acid sequence that does not exist in nature. Further, in the present application, the "artificial sequence peptide" is also simply referred to as an "artificial peptide".

The present application provides compositions containing a specific artificial sequence peptide for use in mobilizing mesenchymal stem cells into peripheral blood.

The compositions for use in mobilizing mesenchymal stem cells into the peripheral blood of the present application can be used as a pharmaceutical composition or a reagent composition. In the present application, the term "pharmaceutical composition" is used interchangeably with "medicament", "drug" or "pharmacological composition", and the term "reagent composition" is used interchangeably with "reagent".

The compositions for use in mobilizing mesenchymal stem cells into peripheral blood of the present application can be used for treating a disease or pathological condition in a subject, for example, by mobilizing mesenchymal stem cells into peripheral blood.

Mesenchymal stem cells mobilized into peripheral blood using the composition for mobilizing mesenchymal stem cells into the peripheral blood of the present application can also be collected from the body, concentrated, and then used for treatment of a disease or pathological condition in a subject. The present application also provides use of a specific artificial sequence peptide in the manufacture of a medicament or a reagent for collecting mesenchymal stem cells from the body.

The compositions for use in mobilizing mesenchymal stem cells into the peripheral blood of the present application can also be used in, for example, basic research, clinical research and such. Basic research and clinical research include, but are not limited to, mesenchymal stem cell mobilization research in vitro and mesenchymal stem cell mobilization research in laboratory animals. The present application also provides use of specific artificial sequence peptides in the manufacture of pharmaceuticals or reagents for basic or clinical research.

The compositions for use in mobilizing mesenchymal stem cells into peripheral blood can comprise one or more artificial sequence peptides.

In the present application, "mesenchymal stem cells" are cells that are collected from bone marrow or other tissues (blood such as umbilical cord blood, and skin, fat, pulp, etc.), can be cultured and proliferated on culture dishes (plastic or glass) as adherent cells, and have the ability to differentiate into mesenchymal tissues such as bone, cartilage, fat, and muscle. In one embodiment, mesenchymal stem cells also have the ability to differentiate into epithelial tissues and nerve tissues. In one embodiment, mesenchymal stem cells are cells capable of forming colonies. In the present application, mesenchymal stem cells may exist as a heterogeneous cell population comprising not only stem cells in the narrow sense (cells having self-renewal ability and differentiation ability) but also progenitor cells. Under culture conditions, the mesenchymal stem cells may include stem cells in the narrow sense, or may even include differentiated cells in addition to stem cells in the narrow sense and progenitor cells. In one embodiment, the mesenchymal stem cells may be composed only of stem cells in the narrow sense.

In the present application, progenitor cells are defined as cells with a unidirectional ability to differentiate into cells of specific tissues other than the blood system, and include cells that have the ability to differentiate into mesenchymal tissues, epithelial tissues, nerve tissues, parenchymatous organs, vascular endothelium.

In the present application, the mesenchymal stem cells include, but are not limited to, bone marrow mesenchymal stem cells and bone marrow-derived mesenchymal stem cells. The "bone marrow mesenchymal stem cells" exist in the bone marrow, and may be harvested from bone marrow and cultured and proliferated as adherent cells on culture dish (made of plastic or glass); and they are cells characterized in having the ability to differentiate into mesenchymal tissues such as bone, cartilage, fat, muscle and such. In one embodiment, bone marrow mesenchymal stem cells also have the ability to differentiate into epithelial tissues and nerve tissues. In one embodiment, bone marrow mesenchymal stem cells are cells capable of forming colonies. In the present application, the term "bone marrow mesenchymal stem cell" is used interchangeably with "bone marrow mesenchymal stromal cell", "bone marrow pluripotent stem cell" or "bone marrow pluripotent stromal cell".

"Bone marrow-derived mesenchymal stem cells" refers to bone marrow mesenchymal stem cells that have been mobilized from bone marrow to the outside of the bone marrow, and are cells that can be collected by peripheral blood collection, and further from mesenchymal tissues such as fat, epithelial tissues such as skin, or nerve tissues such as the brain. In the present application, the term "bone marrow-derived mesenchymal stem cell" can be used interchangeably with "bone marrow-derived mesenchymal stromal cell", "bone marrow-derived pluripotent stem cell" or "bone marrow-derived pluripotent stromal cell".

In one embodiment, bone marrow mesenchymal stem cells and bone marrow-derived mesenchymal stem cells are also characterized in that, by being administered to an injured part of a living body directly after collection or after once attached to a culture dish, the cells are also capable of differentiating into, for example, epithelial tissues such as skin-constituting keratinocytes or tissues of the nerve system which constitutes the brain.

Bone marrow mesenchymal stem cells and bone marrow-derived mesenchymal stem cells preferably have the ability to differentiate into osteoblast cells (identifiable by calcium deposition observed when differentiation is induced), cartilage cells (identifiable by being Alcian blue staining-positive, safranin-O staining-positive, or such), and fat cells (identifiable by being Sudan III staining-positive or such), and also differentiate into, for example, mesenchymal cells such as fibroblasts, smooth muscle cells, skeletal muscle cells, stromal cells, and tendon cells, nerve cells, pigment cells, epidermal cells, hair follicle cells (expressing cytokeratin family, hair keratin family or such), epithelial cells (for example, epithelial keratinized cells and intestinal epithelial cells express cytokeratin family or such), endothelial cells, and further differentiate into cells of parenchymal organs such as liver, kidney and pancreas, but the differentiated cells are not limited to the above cells.

Human mesenchymal stem cell markers can be exemplified by some or all of PDGFRα positive, PDGFRβ positive, Lin negative, CD45 negative, CD44 positive, CD90 positive, CD29 positive, Flk-1 negative, CD105 positive, CD73 positive, CD90 positive, CD71 positive, Stro-1 positive, CD106 positive, CD166 positive, CD31 negative, CD271 positive, and CD11b negative, but are not limited thereto.

Murine mesenchymal stem cell markers can be exemplified by some or all of CD44 positive, PDGFRα positive, PDGFRβ positive, CD45 negative, Lin negative, Sca-1 positive, c-kit negative, CD90 positive, CD105 positive, CD29 positive, Flk-1 negative, CD271 positive, and CD11b negative, but are not limited thereto.

Rat mesenchymal stem cell markers can be exemplified by some or all of PDGFRα positive, CD44 positive, CD54 positive, CD73 positive, CD90 positive, CD105 positive, CD29 positive, CD271 positive, CD31 negative, and CD45 negative, but are not limited thereto.

In the present application, examples of mesenchymal stem cells include PDGFRα-positive mesenchymal stem cells, PDGFRα-positive bone marrow-derived mesenchymal stem cells, and PDGFRα-positive bone marrow-derived cells obtained as adherent cells by cell culture of a mononuclear cell fraction in blood obtained by bone marrow harvest (bone marrow cell collection) or peripheral blood collection, but they are not limited thereto. Examples of PDGFRα-positive mesenchymal stem cells include PDGFRα- and CD44-positive cells, PDGFRα- and CD90-positive cells, PDGFRα- and CD105-positive cells, PDGFRα- and CD29-positive cells, and such. In one embodiment, PDGFRα-positive mesenchymal stem cells may be CD44-negative cells.

The present application provides compositions for use in the treatment of a disease or a pathological condition in a subject by mobilizing mesenchymal stem cells into the peripheral blood, comprising a specific artificial sequence peptide.

The compositions for use in the treatment of a disease or a pathological condition in a subject by mobilizing mesenchymal stem cells into the peripheral blood in the present application can be used as pharmaceutical compositions.

The subject in the present application is not particularly limited, and examples thereof include mammals, birds, and fish. Mammals include human and non-human animals, for example, human, mouse, rat, monkey, pig, dog, rabbit, hamster, guinea pig, horse, sheep, and whale, but are not limited thereto. In the present application, the term "subject" is used interchangeably with "patient", "individual", or "animal".

The composition for use in the treatment of a disease or pathological condition in a subject by mobilization of mesenchymal stem cells into the peripheral blood in the present application can comprise one or more artificial sequence peptides.

In the present application, the treatment of a disease or pathological condition is selected from, for example, inflammation-suppressing therapy, immunomodulatory therapy, tissue regeneration-inducing therapy, and tissue fibrosis-suppressing therapy, but is not limited thereto.

In the present application, the disease or pathological condition is selected from inflammatory diseases, autoimmune diseases, diseases accompanied by tissue damage, ischemia, or necrosis, and fibrotic diseases, but is not limited thereto.

In the present application, the inflammatory disease includes, for example, inflammatory bowel disease and atopic dermatitis, but is not limited thereto. The autoimmune disease includes, for example, inflammatory bowel disease, but is not limited thereto. The fibrotic disease includes, for example, lung fibrosis, but is not limited thereto. The disease accompanied by tissue damage, ischemia, or necrosis includes, for example, inflammatory bowel disease and cerebral infarction, but is not limited thereto. The inflammatory bowel disease includes, but is not limited to, ulcerative colitis and Crohn's disease.

In the present application, the term "activity of mobilizing mesenchymal stem cells into peripheral blood" is used interchangeably with "activity to increase the abundance of mesenchymal stem cells in peripheral blood".

The activity of an artificial sequence peptide in the present application to mobilize mesenchymal stem cells into the peripheral blood can be assessed by i) collecting peripheral blood from an individual administered with an artificial sequence peptide and an individual not administered with the artificial sequence peptide, seeding and culturing in a culture dish (several days to 10 days), and counting the number of colonies formed; and ii) confirming that the formed colonies have the ability to adhere to the solid phase and proliferate (self-renewal ability), and the ability to differentiate into osteoblasts, chondrocytes and adipocytes. In i) above, before seeding the collected peripheral blood on a culture dish, red blood cells may be removed from the peripheral blood in a desired manner.

The artificial sequence peptide in the present application can be obtained as a recombinant by incorporating DNA encoding it into an appropriate expression system, or it can be artificially synthesized. Thus, artificial sequence peptides in this application include peptides prepared using cells, and artificially synthesized peptides (i.e., synthetic peptides).

In order to obtain the artificial sequence peptide in the present application by genetic engineering techniques, DNA encoding the peptide may be incorporated into an appropriate expression system and expressed.

Hosts applicable to the present application include, but are not limited to, prokaryotic cells and eukaryotic cells. In addition, hosts applicable to the present application also include bacteria (e.g., *Escherichia coli*), yeast, animal cells (e.g., mammalian cells such as HEK293 cells and CHO cells, insect cells such as silkworm cells), plant cells and such, but are not limited thereto.

As the host/vector system applicable to the present application, for example, the expression vector pGEX and *Escherichia coli* can be shown. pGEX can express a foreign gene as a fusion protein with glutathione S-transferase (GST) (Gene, 67: 31-40, 1988). As such, pGEX into which DNA encoding the artificial sequence peptide of the present application has been incorporated is introduced into an *E. coli* strain such as BL21 by heat shock, and after culturing for an appropriate period of time, isopropylthio-β-D-galactoside (IPTG) is added to induce the expression of the GST fusion peptide. GST in the present application adsorbs to glutathione Sepharose 4B, and thus the expression product can be easily separated and purified by affinity chromatography.

In addition to this, the following can be applied as a host/vector system for obtaining a genetic recombinant of the artificial sequence peptide of the present application. First, when a bacterium is used as a host, a vector for expressing a fusion protein using a tag or such is commercially available. In addition, the genetic recombinant of the present application also includes those in which a tag or a partial peptide thereof is added.

The tag added to the artificial sequence peptide of the present application is not particularly limited as long as it does not affect the activity of the artificial sequence peptide of the present application, and includes, for example, a histidine tag (for example, 6x His, 10x His), HA tag, FLAG tag, GST tag, T7-tag, HSV-tag, E-tag, lck tag, and B-tag.

Among yeasts, it is known that Pichia yeast is effective for the expression of proteins having sugar chains. In terms of the addition of sugar chains, the expression system that uses a Baculovirus vector, which uses an insect cell as a host, is also useful (Bio/Technology, 6:47-55, 1988). Furthermore, mammalian cells are used for transfection with a vector that uses the promoter of CMV, RSV, SV40, or such. These host/vector systems can be used as an expression system for the artificial sequence peptide of the present application. In addition, plasmid vectors, retrovirus vectors, lentivirus vectors, adenovirus vectors, adeno-associated virus vectors, Sendai virus vectors, Sendai virus envelope vectors, papilloma virus vectors, and such virus vectors may also be used to introduce the gene, without limitation thereto. The vector may also contain a promoter DNA sequence that effectively induces gene expression, factors that control gene expression, and molecules necessary to maintain the stability of the DNA.

The resulting artificial sequence peptide in the present application can be isolated from the host cell or outside of the cell (such as medium), and purified as a substantially pure homogeneous peptide. For separation and purification of peptides, any separation and purification methods used in standard peptide purification may be utilized, without limitation. For example, chromatography columns, filters, ultrafiltration, salting out, solvent precipitation, solvent extraction, distillation, immunoprecipitation, SDS-polyacrylamide gel electrophoresis, isoelectric focusing, dialysis, recrystallization, and such are appropriately selected and combined for peptide separation and purification.

Chromatography includes, for example, affinity chromatography, ion exchange chromatography, hydrophobic chromatography, gel filtration, reverse phase chromatography, adsorption chromatography and such (Marshak et al, Strategies for Protein Purification and Characterization: A Laboratory Course Manual. Ed Daniel R. Cold Spring Harbor Laboratory Press, 1996). These chromatographies can be performed using liquid chromatography such as HPLC and FPLC.

Further, the artificial sequence peptide in the present application is preferably a substantially purified peptide. Here, "substantially purified" means that the degree of purification of the artificial sequence peptide of the present application (the ratio of the artificial sequence peptide of the present application in the entire protein component) is 50% or more, 60% or more, 70% or more, 80% or more, 90% or more, 95% or more, 100% or nearly 100%. The nearly 100% upper limit depends on the purification techniques and analysis techniques of those skilled in the art and may be, for example, 99.999 %, 99.99 %, 99.9 %, or 99%.

Further, the substantially purified artificial sequence peptide includes those purified by whatever purification method as long as they have the above purity. Examples include, but are not limited to, artificial sequence peptides substantially purified by appropriately selecting or combining the above-mentioned chromatography columns, filters, ultrafiltration, salting out, solvent precipitation, solvent extraction, distillation, immunoprecipitation, SDS-polyacrylamide gel electrophoresis, isoelectric focusing, dialysis, recrystallization and such.

On the other hand, the artificial sequence peptides in the present application can also be artificially synthesized. In the peptide synthesis method of the present application, peptides can be chemically synthesized by methods such as a peptide liquid-phase synthesis method and a peptide solid-phase synthesis method. The peptide solid-phase synthesis method is one of the methods generally used when chemically synthesizing a peptide. Polystyrene polymer gel beads having a diameter of about 0.1 mm modified with amino groups on the surface are used as a solid phase, from which the amino acid chain is extended one by one via dehydration reaction. When the sequence of the target peptide is completed, it is excised from the solid phase surface to obtain the target substance. Solid phase synthesis enables synthesis of ribosome peptides, which are difficult to synthesize in bacteria, introduction of unnatural amino acids such as D-form and stable isotope ($^2$H, $^{13}$C, $^{15}$N, etc.)-substituted amino acids, introduction of heavy atom-substituted amino acids (e.g., selenoamino acids such as selenomethionine), modification of peptide and protein main chains, and such. When synthesizing a long peptide chain of 70 to more than 100 amino acids in the solid phase method, it can be synthesized by ligating two peptide chains using the native chemical ligation method. The artificial sequence peptide in the present application may be in the form of a pharmaceutically acceptable salt of the peptide. Examples of pharmaceutically acceptable salts include, but are not limited to, hydrochlorides, acetates, and trifluoroacetates. The artificial sequence peptide in the present application may be in the form of a solvate of the peptide, or a solvate of a pharmaceutically acceptable salt of the peptide. A solvate refers to a solute molecule to which an arbitrary number of solvent molecules are coordinated, and examples thereof include hydrates, but are not limited thereto.

The amino acid length of the artificial sequence peptide in the present application includes, for example, 25 to 35 amino acids, 20 to 40 amino acids, 10 to 50 amino acids, 10 to 70 amino acids, and 10 to 100 amino acids, but is not limited thereto.

Examples of the artificial sequence peptide in the present application include peptides selected from below:

(i) an artificial sequence peptide comprising the amino acid sequence of SEQ ID NO: 1;
(ii) an artificial sequence peptide consisting of the amino acid sequence of SEQ ID NO: 1;
(iii) an artificial sequence peptide consisting of a portion of the amino acid sequence of SEQ ID NO: 1;
(iv) an artificial sequence peptide comprising an amino acid sequence resulting from substitution, deletion, insertion, or addition of one or several amino acids in the amino acid sequence of SEQ ID NO: 1;
(v) an artificial sequence peptide consisting of an amino acid sequence resulting from substitution, deletion, insertion, or addition of one or several amino acids in the amino acid sequence of SEQ ID NO: 1;
(vi) an artificial sequence peptide comprising an amino acid sequence having about 90% or more sequence identity with the amino acid sequence of SEQ ID NO: 1;
(vii) an artificial sequence peptide consisting of an amino acid sequence having about 90% or more sequence identity with the amino acid sequence of SEQ ID NO: 1;
(viii) an artificial sequence peptide encoded by a DNA consisting of the nucleotide sequence of SEQ ID NO: 2; and
(ix) an artificial sequence peptide encoded by a DNA that hybridizes under stringent conditions with a DNA consisting of the nucleotide sequence of SEQ ID NO: 2.

In the present application, "several" includes, for example, 1 to 5, 1 to 4, 1 to 3, or 1 or 2.

In the present application, "about 90% or more" means, for example, about 90% or more, about 91% or more, about 92% or more, about 93% or more, about 94% or more, about 95% or more, about 96% or more, about 97% or more, about 98% or more, or about 99% or more.

In the present application, the "stringent conditions" can be shown as conditions of, for example, hybridization at $6\times$ SSC, 40% formamide, 25°C, and washing at $1\times$ SSC, 55°C. Stringency is affected by conditions such as salt concentration, formamide concentration, or temperature, and those skilled in the art can set these conditions to obtain the required stringency.

When the hybridization is carried out under stringent conditions, a DNA having a high homology in terms of nucleotide sequence is selected, and the possibility is increased for the peptide isolated as a result to comprise a peptide functionally equivalent to a peptide consisting of the amino acid sequence of SEQ ID NO: 1. A nucleotide sequence having high homology can exhibit, for example, about 60% or more, about 70% or more, or about 80% or more identity. Herein, "functionally equivalent" means equivalence in the function concerning an activity of mobilizing mesenchymal stem cells into peripheral blood.

The artificial sequence peptide selected from (i) to (ix) above is an artificial sequence peptide having an activity of mobilizing mesenchymal stem cells into peripheral blood. Thus, these artificial sequence peptides are thought to have the effect of mobilizing mesenchymal stem cells into peripheral blood, as well as therapeutic effects on inflammatory diseases, autoimmune diseases, diseases accompanied by tissue damage, ischemia, or necrosis, and fibrotic diseases.

The present application also provides an artificial sequence peptide selected from (i) to (ix) above.

The amino acid sequence described in SEQ ID NO: 1 is the amino acid sequence of the artificial sequence peptide 1r10 of the present application.

The nucleotide sequence described in SEQ ID NO: 2 is an example of the nucleotide sequence of DNA encoding the artificial sequence peptide 1r10 of the present application. Other DNA sequences encoding the artificial sequence peptide 1r10 of the present application can be produced by a method of converting the amino acid residues of the artificial sequence peptide 1r10 to corresponding codons using codon tables known to those skilled in the art (reverse translation). Reverse translation can be performed by using a variety of software (including programs, algorithms, etc.) developed for the analysis of amino acid and nucleic acid sequences as desired.

An effective amount of an artificial sequence peptide of the present application, or a pharmaceutical composition comprising the same (hereinafter referred to as a pharmaceutical composition and such) is administered to a subject for the treatment of diseases and conditions described herein.

The effective amount in the present application means an amount sufficient for the treatment of the diseases or pathological conditions described herein. The treatment in the present application includes alleviation, delay, inhibition, amelioration, remission, cure, and full recovery, but are not limited thereto.

There is no limitation on the site of administration of the pharmaceutical composition and such of the present application, and the pharmaceutical composition and such of the present application can exert its effect when administered to any site, such as a site where the symptoms of the disease or pathological condition appear or a site nearby, a site different from these sites (sites other than these sites), a site separated from a site where the symptoms of the disease or pathological condition appear, a site distal to a site where the symptoms of the disease or pathological condition appear, or a site distal and ectopic to a site where the symptoms of the disease or pathological condition appear.

Further, the pharmaceutical composition and such of the present application can exert its effect when administered to any tissue, such as a tissue different from a tissue in which the symptoms of the disease or the pathological condition appear, a tissue separated from a tissue in which the symptoms of the disease or the pathological condition appear, a tissue distal to a tissue in which the symptoms of the disease or the pathological condition appear, or a tissue distal and ectopic to a tissue in which the symptoms of the disease or pathological condition appear.

Methods of administering the pharmaceutical composition and such of the present application include, but are not limited to, oral administration and parenteral administration, and methods of parenteral administration include intravascular administration (intra-arterial administration, intravenous administration, etc.), intramuscular administration, subcutaneous administration, intradermal administration, intraperitoneal administration, nasal administration, pulmonary administration, transdermal administration, and such. In addition, the pharmaceutical composition and such of the present application can be administered systemically or locally (for example, subcutaneously, intradermally, or to the skin surface, eyeball, or palpebral conjunctiva, nasal mucosa, oral and gastrointestinal mucosa, vaginal and endometrial mucosa, or injured site) by injection administration, for example, intravenous injection, intramuscular injection, intraperitoneal injection, and subcutaneous injection.

In place of the artificial sequence peptide of the present application, a cell secreting the artificial sequence peptide, a gene therapy vector into which a DNA encoding the artificial sequence peptide is inserted, and a pharmaceutical composition comprising them can be used.

In addition, the administration method can be appropriately selected depending on the age and symptoms of a patient. When the pharmaceutical composition and such of the present application is administered, for example, the dose can be selected from the range of 0.0000001 mg to 1000 mg per kilogram of body weight per administration. Alternatively, for example, the dose can be selected from the range of 0.00001 to 100000 mg/body per patient. When administering cells secreting the artificial sequence peptide of the present application or gene therapy vectors into which DNA encoding the artificial sequence peptide is inserted, they can be administered so that the amount of the artificial sequence peptide is within the above range. However, the pharmaceutical compositions in the present application are not limited to these dosages.

The pharmaceutical compositions of the present application can be formulated according to conventional methods (e.g., Remington's Pharmaceutical Science, latest edition, Mark Publishing Company, Easton, U.S.A.), and may contain pharmaceutically acceptable carriers or additives together. Examples include, but are not limited to, surfactants, excipients, coloring agents, perfumes, preservatives, stabilizers, buffers, suspending agents, isotonizing agents, binding agents, disintegrants, lubricants, fluidity-promoting agents, and flavoring agents. Other commonly used carriers can also be used as appropriate. Specific examples include light anhydrous silicic acid, lactose, crystalline cellulose, mannitol, starch, carmellose calcium, carmellose sodium, hydroxypropyl cellulose, hydroxypropyl methyl cellulose, polyvinylacetal diethylaminoacetate, polyvinylpyrrolidone, gelatin, medium-chain fatty acid triglyceride, polyoxyethylene hydrogenated castor oil 60, white sugar, carboxymethyl cellulose, cornstarch, and inorganic salts.

All prior art documents cited herein are incorporated herein as references.

Herein below, the present invention will be further illustrated with reference to Examples, but it is not to be construed as being limited thereto.

[Examples]

Example 1

Mobilization of mesenchymal stem cells by artificial sequence peptide

(1) Materials and methods

i) Peptide production

An artificial sequence peptide consisting of the amino acid sequence of SEQ ID NO: 1 was chemically synthesized (the obtained peptide was in the form of a trifluoroacetic acid (TFA) salt). Hereinafter, the peptide is referred to as "artificial sequence peptide 1r10" or "peptide 1r10".

ii) Peptide administration

C57BL/6J mice (8 weeks old, male, body weight 25 g) were prepared and divided into a group administered with the artificial sequence peptide 1r10 and a control group. The peptide was administered by injecting into the tail vein, a solution of peptide 1r10 adjusted to a concentration of 1 $\mu$g/$\mu$L using physiological saline as a solvent in an amount of 100 $\mu$L/animal (4 mg/kg dose of peptide). For the control group, physiological saline was injected into the tail vein in an amount of 100 $\mu$L/animal.

iii) Cell collection from peripheral blood

Fourteen hours after the administration of physiological saline or the artificial sequence peptide 1r10, about 800 $\mu$L to 1000 $\mu$L of peripheral blood was collected from the hearts under general anesthesia (1 mL syringe containing heparin was used). To remove red blood cells, an equal volume of Hetasep (STEMCELL Technologies Inc., Cat No. ST-07906) as the collected blood was added and centrifuged for 2 min at 100G, incubated for 15 min at room temperature, and then the supernatant was collected. The supernatant was used in the next experiment as a sample containing nucleated cells in peripheral blood.

iv) Colony assay

The supernatant (sample containing peripheral blood-derived cells) obtained by the above procedure was seeded on collagen I-coated 6-well plates (Corning, Cat No. 356400), and cultured for 10 days under the conditions of 37°C, 5% $CO_2$ and 5% $O_2$, using a medium containing Expansion Medium prepared using the MesenCult Expansion Kit (STEMCELL Technologies, Cat No. ST-05513) according to the manual of the kit, 1% L-glutamine (Nacalai Tesque Inc.), 10 $\mu$M ROCK inhibitor (Y27632, Tocris Bioscience) and 1% penicillin/streptomycin (Nacalai Tesque Inc.) (all numerical values are final concentrations). The medium was replaced with fresh medium twice a week during the culture period. On the 10th day of culture, cells on the plates were stained with a Differential Quik Stain Kit (Sysmex Corporation, Cat No. 16920), and the number of colonies containing 50 or more cells was counted.

In the experiments conducted by the present inventors so far, all the colonies obtained as a result of culturing peripheral blood on a solid phase such as a dish or a plate have adherability to the solid phase and have self-renewal ability. In addition, they have been confirmed to be PDGFR$\alpha$ positive, and have the ability to differentiate into bone, cartilage, fat, epithelium, and such.

In addition, the colonies obtained as a result of culturing, on a solid phase, peripheral blood after administration of a peptide consisting of amino acid residues 1-44 of the human HMGB1 protein (hereinafter, HMGB1 peptide 1-44), which has an activity of mobilizing mesenchymal stem cells into peripheral blood, have been confirmed to have adherability to solid phase and self-renewal ability, and to be PDGFR$\alpha$ positive. They have also been confirmed to have a gene expression profile characteristic to mesenchymal stem cells, based on the results of clustering the transcriptome analysis data and performing gene ontology analysis.

Furthermore, it has been confirmed that the number of colonies obtained by solid-phase culture is larger in the peripheral blood after administration of HMGB1 peptide 1-44 than in the peripheral blood after administration of physiological saline.

Therefore, the colonies obtained as a result of culturing peripheral blood on a solid phase are mesenchymal stem cells, and it is considered that an increase in the number of colonies detected in a solid phase culture of peripheral blood indicates an increase in the number of mesenchymal stem cells in the peripheral blood.

In addition, since usually mesenchymal stem cells are rarely present in peripheral blood, it is thought that the increased amount of mesenchymal stem cells was mobilized into peripheral blood from tissues other than peripheral blood (for example, bone marrow).

From the above, the number of colonies detected in the solid-phase culture of peripheral blood after administration of a test substance can be used as an indicator of the activity of the test substance to mobilize mesenchymal stem cells into peripheral blood.

(2) Result

In the mice administered with the peptide 1r10, the number of colonies obtained on the plate by culturing the peripheral blood-derived cells was larger than that in the mice administered with physiological saline (Figure 1).

As described above, an increase in the number of colonies detected by the colony assay described herein indicates an increase in the number of mesenchymal stem cells in peripheral blood, and thus these results demonstrate that the peptide 1r10 has the activity of mobilizing mesenchymal stem cells into peripheral blood.

Example 2

Efficacy of artificial sequence peptide for inflammatory bowel disease

(1) Materials and methods

i) Drug

Dextran sodium sulfate (DSS) (molecular weight 36,000 to 50,000, manufactured by MP Biomedicals, Product No. 160110) was dissolved in purified water to prepare a 2.5% (w/v) DSS aqueous solution. In addition, the peptide 1r10 (TFA salt) described in Example 1 was used as the test substance.

ii) Generation of inflammatory bowel disease (IBD) model mice

BALB/c mice (9 weeks old, male) were allowed to drink the 2.5% DSS aqueous solution freely for 7 days to induce colitis. After that, the drinking was changed from DSS to tap water, and the large intestine was collected 3 days later (10 days after the start of drinking the DSS aqueous solution).

iii) Peptide administration

The IBD model mice prepared as described above were divided into the peptide 1r10 administration group (n = 8) and the control group (n = 8). Peptide administration was carried out by injecting into the tail vein, the peptide 1r10 solution adjusted to a concentration of 0.5 mg/mL using physiological saline as a solvent at an amount of 10 mL/kg on Days 1, 4, 7, 8 and 9 after the start of drinking the DSS aqueous solution (peptide dose of 5 mg/kg). The control group was injected with physiological saline solution at a dose of 10 mL/kg into the tail vein on Days 1, 4, 7, 8 and 9 after the start of drinking the DSS aqueous solution.

iv) Evaluation of the effect of peptide administration

For 10 days from the start of drinking the DSS aqueous solution, the mice were weighed and observed for the symptoms (stool condition and bleeding) every day except Saturdays, Sundays, and holidays, and the degree of symptoms was evaluated by calculating the DAI (disease activity index) score. The DAI score was calculated by scoring the weight loss rate, stool condition, and stool bleeding as shown in the table below, and summing the scores of these three items. The scoring criteria were partly based on the method of Li et al. (PLoS One. 2015 Dec 7; 10 (12): e0144101).

[Table 1]
DAI (disease activity index) scoring

|  | Item | Score |
| --- | --- | --- |
| Rate of weight loss | No reduction | 0 |
|  | 1-5% | 1 |
|  | 5-10% | 2 |
|  | 10-20% | 3 |
|  | >20% | 4 |
| Stool condition | Normal | 0 |
|  | Loose stool | 2 |
|  | Diarrhea or watery stool | 4 |
| Bleeding | Absent | 0 |
|  | Bloody stool | 1 |
|  | Bloody stool (overall) | 2 |
|  | Bleeding from the rectum | 4 |

In addition, the length of the large intestine (from the colon to the rectum) collected 10 days after the start of drinking DSS was measured using a digital caliper.

(2) Result

Figure 2 shows the changes in mouse body weight during the test period (see "saline" for the control group and "1r10" for the peptide-administered group). Both the control group and the peptide-administered group lost their body weight over time after the start of drinking DSS, but the degree of decrease was slower in the peptide-administered group than in the control group, and 10 days after the start of drinking the DSS aqueous solution, the peptide-administered group showed a tendency to recover body weight.

The stool score (sum of the "stool condition" score and the "bleeding" score scored as shown in Table 1 above) and the DAI score 8 to 10 days after the start of drinking the DSS aqueous solution are shown in Figures 3 and 4, respectively (see "saline" for the control group and "1r10" for the peptide-administered group). Both the stool score and the DAI score were lower in the peptide-administered group than in the control group.

Ten days after the start of drinking the DSS aqueous solution, the colon length of the peptide-administered group was longer than that of the control group (Figure 5; see "saline" for the control group and "1r10" for the peptide-administered group).

In IBD, inflammation occurs in the mucous membrane of the intestinal tract, erosions and ulcers are formed, and symptoms such as narrowing and shortening of the intestinal tract may occur. Another symptom of IBD is known to be occurrence of body weight loss, and the reason is thought to be malnutrition due to inflammation and tissue damage (such as ulcer) that occur in the intestinal mucosa. Furthermore, intravenous injection of mesenchymal stem cells in an animal IBD model is known to improve various symptoms including body weight loss, epithelial damage in the intestinal tract, infiltration of inflammatory cells, and such. Such improvement of symptoms is due to suppression of inflammation of the intestinal mucosa by the anti-inflammatory effect of mesenchymal stem cells, and resulting facilitation of mucosal tissue regeneration, and such.

This time, by administering the artificial sequence peptide of the present application to the IBD model mice, body weight loss was suppressed, reduction of colon length was suppressed, and DAI score was improved. This is considered to be a result of the mesenchymal stem cells being mobilized into the peripheral blood by the action of the artificial sequence peptide of the present application, and the cells exerting the anti-inflammatory effect and the tissue regeneration effect.

Example 3

Efficacy of the artificial sequence peptide for atopic dermatitis

(1) Materials and methods

i) Drug

MC903 (generic name: Calcipotriol) was used to induce atopic dermatitis (hereinafter, also referred to as "AD") in mice. In addition, the peptide 1r10 (TFA salt) described in Example 1 was used as the test substance.

ii) Generation of atopic dermatitis model mice

C57BL/6 mice (C57BL/6JJcl, 7-8 weeks old, male, microbial grade SPF) were obtained from Japan Claire Co., Ltd., acclimated in the animal breeding room for 5 days or more, and then subjected to the following experiments. An ethanol-adjusted MC903 solution was applied to the skin of the auricle of the mice at a dose of 2.25 nmol MC903/ear/once/day five times a week for 2 weeks (ten times in total) to induce atopic dermatitis (AD). This MC903-induced AD model is a model in which AD-like pathology can be reproduced in a short period of time (1-2 weeks) by artificially inducing the secretion of TSLP from epithelial cells, which is actually seen in AD patients, thereby eliciting a series of type 2 immune response cascades (Li M et al., Proc Natl Acad Sci USA. 2006; 103:11736-41.). In addition, as a preliminary test, the appearance and tissue of the auricle were observed in normal mice and MC903-induced AD mice (14 days after the start of induction), and as a result the induced AD mice were found to develop erythema due to inflammation, scaling in the marginal area, and keratinous lichenization, epidermal acanthosis (thickening of the epidermis due to spinous cell proliferation), destruction of local barriers, infiltration of immune cells into the dermis, increased dermis thickness, and spongiosis in parts of the epidermis.

iii) Peptide administration

Two weeks (Day 15) after the start of application of MC903 (referred to as Day 1), only individuals in which AD was clearly induced judging from the main symptoms of scaling, erythema, and edema were selected. They were then divided into the control group and the peptide administration group (n = 7 in each group), while the groups were equalized based on the auricle thickness in consideration of the difference in the severity of inflammation between individuals. For peptide administration, the solution of peptide 1r10 adjusted to 0.3 mg/mL with physiological saline was injected into the tail vein at a volume of 5 mL/kg (peptide dose of 1.5 mg/kg) twice in total, once on the grouping day (Day 15) and once during the following 4 days (Day 16 to Day 19). For the control group a solution of bovine serum albumin (BSA) adjusted to 0.3 mg/mL with physiological saline was injected into the tail vein at a volume of 5 mL/kg (BSA dose of 1.5 mg/kg) twice in total, once on the grouping day (Day 15) and once during the following 4 days (Day 16 to Day 19).

iv) Evaluation of the effect of peptide administration

Evaluation of edema and scaling

During the peptide administration period (Day 15 to Day 19), ear swellings were measured daily to determine edema. Scaling, which is the main symptom of AD, was determined by photography (three times a week) using a digital camera (E-M1 II; Olympus) from a fixed base at a height of 13-14 cm from the auricle.

Pathological analysis

On the 4th day (Day 19) after the start of peptide administration, mice were placed under the influence of isoflurane anesthesia and euthanized with cervical spine dislocation. The skin at auricular inflammation site and its surroundings was quickly excised and collected while on ice to prevent protein denaturation of the sample. After removing excess hair and skin with tweezers, the collected auricular skin was immersed in 10% formalin and fixed at 4°C overnight while shaking on ice using a shaker. After fixation, the skin was thoroughly washed with phosphate buffered saline (PBS) and trimmed, and then a paraffin block was made using an automatic embedding device (Excelsior ES; Thermo Scientific) with attention to the tissue polarity in the dorsoventral axis/left-right axis specific to the skin). Then, it was sliced (5 $\mu$m thick) with a rotary microtome (HM 325; Thermo Scientific), fixed on a slide glass, and subjected to various staining. As pathological analysis, skin thickness and infiltration of immune cells were evaluated by histochemical staining and fluorescent immunostaining, respectively.

Measurement of dermis thickness

To quantitatively evaluate the healing condition of the site of inflammation, hematoxylin-eosin (HE) staining of the paraffin thin sections (5 $\mu$m thick) of skin tissue generated as described above was performed to measure the dermal thickness. Specifically, three sections were prepared for each individual, and each section was photographed with a microscope (Keyence BZ-X710) at one site each of the center of inflammation, head side, and tail side (that is, a total of 9 sites were photographed per individual). Next, for the photographed image, perpendicular lines were drawn from the epidermis-dermis junction to the subcutaneous fat-cartilage junction, and only the longest perpendicular line in the visual field was selected and used as the measured value of dermis thickness.

Quantification of immune cells

In order to quantitatively evaluate the number of immune cells at the site of inflammation, fluorescent immunostaining was performed on the paraffin slice (5 $\mu$m thick) prepared as described above using an anti-Laminin antibody and anti-CD45 antibody (for the detection of Laminin, Anti-Laminin (Sigma Aldrich; Catalog No. L9393) was used as the primary antibody, and Donkey anti-Rabbit IgG (H + L), Alexa Fluor 488 (Thermo Fisher Scientific; Catalog No. A-21206) was used as the secondary antibody. For the detection of CD45, Goat Anti-mouse CD45 (R&D Systems; Catalog No. AF114) was used as the primary antibody and Cy3 AffiniPure Bovine Anti-Goat IgG (H + L) (Jackson ImmunoReseach; Catalog No. 805-165-180) was used as the secondary antibody. For details of quantification, three sections were prepared for each individual, and each section was photographed using a microscope (Keyence BZ-X710) under high-power field (HPF: 400x) at one site each of the center of inflammation, head side, and tail side (that is, a total of 9 sites were photographed per individual). Next, for the photographed image, the CD45-positive cells (detected by Cy3 fluorescence) in the view field were counted and used as the measured value.

Statistical processing

The measured values obtained by the above analysis or the calculated values after correction were analyzed / judged after each statistical value was calculated using statistical analysis software (statcel-3 (OMS Publishing) and Excel statistics (BellCurve)). Specifically, as a preliminary test, (1) normality test (D'Agostino test with skewness/kurtosis coefficient; P> 0.05 is regarded as a normal distribution) and (2) rejection test (Grubbs test; P <0.05 is regarded as an outlier) were performed; and when the result obtained in (1) showed a normal distribution, t-test was performed between the groups. When the result did not show a normal distribution, Mann-Whitney U-test was performed as a nonparametric test based on the ranks between the groups, and a significant difference was determined to be present if p <0.05. For the changes in the body weight and auricle thickness, which were mainly measured repeatedly in time series, the repeated one-way ANOVA test was performed and the Tukey-Kramer test was performed as a post hoc test. Regarding the processing of outliers, the following method was employed: (a) if the number of individuals in the group with P <0.05 in the Grubbs test was 10% or less of the total number of individuals (n) in the group, the outliers were treated as missing values, and (b) if the number of individuals with P <0.05 exceeded 10% of the number n, they were imputed with the mean value of the other individuals with no outliers (so-called mean substitution).

(2) Result

Scaling

In the auricle of the control group mice after the induction of the AD model, scaling due to exfoliated epidermis caused by eczema and dryness, which are typical AD images, frequently occurred at the marginal part (Figure 6, arrow part in the left photograph).). On the other hand, in the peptide-administered group, reduction of scaling in the auricle was observed (Figure 6, arrow part in the right photograph).

Auricular thickness (edema)

The transition of auricular thickness (edema) during the peptide administration period is shown in Figure 7 (see "Control" for the control group and "1r10" for the peptide administration group). A statistically significant reduction in auricular thickness was observed in the peptide-administered group as compared with the control group.

Dermis thickness

As a result of HE staining of skin tissue sections, in the control group, an increase in dermis thickness not seen in the AD non-induced mice (possibly due to cell infiltration and increased vascular permeability associated with the immune response caused by AD) was observed (Figure 8; see "Control" for the control group and "1r10" for the peptide-administered group). On the other hand, in the peptide-administered group, a statistically significant decrease in dermis thickness was observed as compared with the control group (Figure 9; see "Control" for the control group and "1r10" for the peptide-administered group). In addition, when peptide 1r10 was administered at a dose of 0.5 mg/kg, a statistically significant decrease in dermis thickness was observed as compared with the control group (data not shown).

Infiltration of immune cells

As a result of fluorescent immunostaining on skin tissue sections, in the control group, the tissue infiltration of CD45-positive immune cells associated with the immune response caused by AD was observed throughout the skin including around blood vessels (labeled with Laminin) (the arrow part in Figure 10; see "Control" for the control group and "1r10" for the peptide-administered group). On the other hand, in the peptide-administered group, a statistically significant decrease in the number of CD45-positive cells was observed as compared with the control group (Figure 11; see "Control" for the control group and "1r10" for the peptide-administered group).

The changes in mouse body weight during the period of this study (from the start of administration (Day 15) to the time of skin collection (Day 19)) were not significantly different between the groups and statistically significant changes were not observed between the groups (p> 0.05, repeated one-way ANOVA).

In this Example, administration of the artificial sequence peptide of the present application to atopic dermatitis model mice suppressed scaling and edema, suppressed the increase in dermis thickness, and suppressed the infiltration of CD45-positive cells. This is considered to be a result of the mesenchymal stem cells being mobilized into the peripheral blood by the action of the artificial sequence peptide of the present application and the anti-inflammatory effect exhibited by the cells.

Example 4

Efficacy of the artificial sequence peptide for cerebral infarction

(1) Materials and methods

i) Drug

The peptide 1r10 (TFA salt) described in Example 1 was used as the test substance.

ii) Generation of cerebral infarction model rat

Crl:CD (SD) rats (7 weeks old, male) were obtained from Charles River Laboratories, Japan, and after 6 days of acclimation, they were divided into the control group (n = 8) and the peptide-administered group (n = 8). On the following day of grouping, the rats were fixed in the supine position under 2% isoflurane inhalation anesthesia (anesthesia background: laughing gas:oxygen = 7:3). To regulate body temperature during surgery, a thermometer probe connected to a digital thermometer was inserted into the rectum, and changes in the rectal temperature before and after surgery were monitored. As a result, no decrease in rectal temperature was observed in any of the rats, and thus no heating was performed. The right common carotid artery, right external carotid artery and right internal carotid artery were exposed, and the right common carotid artery and right external carotid artery were ligated with sutures. A No. 4 nylon thread (embolus) pre-coated with silicon and cut to a length of 19 mm was inserted from the bifurcation of the right external carotid artery and the right internal carotid artery to occlude the right middle cerebral artery (MCA). After occlusion of the right MCA, the cervical skin was sutured and the rats were released from anesthesia. Thirty minutes after the right MCA occlusion, flexion of the forelimb contralateral to the occluded side was confirmed. Under inhalation anesthesia of 2% isoflurane, the right common carotid artery, right external carotid artery, and right internal carotid artery were exposed again, and 90 minutes after the right MCA occlusion, the embolus was removed to resume blood flow in the right MCA. After resuming blood flow in the right MCA, the right internal carotid artery was ligated and the cervical skin was sutured, and then the animals were released from anesthesia. Prior to the right MCA occlusion surgery, potassium benzylpenicillin was intramuscularly administered at a dose of 20000 units/kg.

iii) Peptide administration

Peptide administration was performed by injecting into the tail vein, 2 mL/kg of a peptide 1r10 solution adjusted to a concentration of 1 mg/mL using physiological saline solution as a solvent (peptide dose of 2 mg/kg) at 105 minutes and 24 hours after the right MCA occlusion. The control group was injected at the tail vein with 2 mL/kg physiological saline solution 105 minutes and 24 hours after the right MCA occlusion.

iv) Evaluation of the effect of peptide administration

Calculation of cerebral infarction volume ratio

Forty-eight hours after the right MCA occlusion, the animals were decapitated under anesthesia with sodium pentobarbital (50 mg/kg, intraperitoneal administration), and the entire brain was excised to prepare brain sections with a thickness of 2 mm. Brain sections were made at positions where coronal planes at 4 mm anterior to bregma, at 2 mm anterior to bregma, at bregma, at 2 mm posterior to bregma, at 4 mm posterior to bregma and at 6 mm posterior to bregma were obtained, with reference to Paxinos and Watson brain atlas (Paxinos G. and Watson C., The rat brain in stereotaxic coordinates, second edition. Academic Press Inc.; 1986). Brain sections were immersed and stained in 1 w/v% 2,3,5-Triphenyltetrazolium chloride (TTC) solution at room temperature and photographed. The resulting photographs were subjected to image analysis to obtain actual measurements of the cerebral infarction area and cross-sectional area of the brain, and the cerebral infarct volume and the total brain volume from 4 mm anterior to bregma to 6 mm posterior to bregma were calculated using the calculation formula below. In addition, the cerebral infarction volume ratio was calculated from the cerebral infarction volume and the total volume of the brain (cerebral infarction volume ratio (%) = cerebral infarction volume / total brain volume x 100).

$$V = 2\,(a + b + \sqrt{ab}\,)/3 + 2\,(b + c + \sqrt{bc}\,)/3 + 2\,(c + d + \sqrt{cd}\,)/3 + 2\,(d + e + \sqrt{de}\,)/3 + 2\,(e + f + \sqrt{ef}\,)/3$$

$$= 2\,(a + f + \sqrt{ab} + \sqrt{bc} + \sqrt{cd} + \sqrt{de} + \sqrt{ef}\,)/3 + 4\,(b + c + d + e)/3$$

V: Cerebral infarction volume or total brain volume ($mm^3$)
a: Cerebral infarct area or cross-section area of the brain on the cross section of 4 mm anterior to bregma ($mm^2$)
b: Cerebral infarct area or cross-section area of the brain on the cross section of 2 mm anterior to bregma ($mm^2$)
c: Cerebral infarct area or cross-section area of the brain on the cross section of bregma ($mm^2$) d: Cerebral infarct area or cross-section area of the brain on the cross section of 2 mm posterior to bregma ($mm^2$)
e: Cerebral infarct area or cross-section area of the brain on the cross section of 4 mm posterior to bregma ($mm^2$)
f: Cerebral infarct area or cross-section area of the brain on the cross section of 6 mm posterior to bregma ($mm^2$)

(2) Result

The cerebral infarction volume ratios of the control group and the peptide-administered group were 34.1 $\pm$ 1.1% and 27.2 $\pm$ 1.2% (mean $\pm$ standard error), respectively (Figure 12; see "Control" for the control group and "1r10" for the peptide-administered group). The cerebral infarction volume ratio in the peptide-administered group was lower than that in the control group, and a statistically significant difference was observed (Mann-Whitney U test, $p < 0.01$).

In this Example, the effect of reducing the cerebral infarction lesion was obtained by administering the artificial sequence peptide of the present application to a cerebral infarction model rat. This is considered to be a result of the mesenchymal stem cells being mobilized into the peripheral blood by the action of the artificial sequence peptide of the present application, and the cells exerting an anti-inflammatory effect, a trophic effect (secretion of trophic factors), a tissue regeneration effect, and such.

[Industrial applicability]

The artificial sequence peptide of the present application can be used as a therapeutic agent for inflammatory diseases, autoimmune diseases, fibrotic diseases, and diseases accompanied by tissue damage/ischemia/necrosis.

**Claims**

1. A composition for use in mobilizing mesenchymal stem cells to peripheral blood, which comprises a peptide selected from the following:

   (a) a peptide comprising the amino acid sequence of SEQ ID NO: 1;
   (b) a peptide comprising an amino acid sequence resulting from substitution, deletion, insertion, or addition of one or several amino acids in the amino acid sequence of SEQ ID NO: 1; and
   (c) a peptide comprising an amino acid sequence with a sequence identity of about 90% or higher with the amino acid sequence of SEQ ID NO: 1.

2. A composition for use in treatment of a disease or pathological condition in a subject by mobilizing mesenchymal stem cells to peripheral blood, which comprises a peptide selected from the following:

(a) a peptide comprising the amino acid sequence of SEQ ID NO: 1;
(b) a peptide comprising an amino acid sequence resulting from substitution, deletion, insertion, or addition of one or several amino acids in the amino acid sequence of SEQ ID NO: 1; and
(c) a peptide comprising an amino acid sequence with a sequence identity of about 90% or higher with the amino acid sequence of SEQ ID NO: 1.

3. The composition of claim 2, wherein the treatment of a disease or pathological condition is selected from inflammation-suppressing therapy, immunomodulatory therapy, tissue regeneration-inducing therapy, and tissue fibrosis-suppressing therapy.

4. The composition of claim 2, wherein the disease or pathological condition is selected from an inflammatory disease, an autoimmune disease, a disease accompanied by tissue damage, ischemia, or necrosis, and a fibrotic disease.

5. The composition of claim 2, wherein the disease or pathological condition is inflammatory bowel disease.

6. The composition of claim 2, wherein the disease or pathological condition is ulcerative colitis.

7. The composition of claim 2, wherein the disease or pathological condition is atopic dermatitis.

8. The composition of claim 2, wherein the disease or pathological condition is cerebral infarction.

9. A composition for use in treatment of a disease selected from inflammatory bowel disease, atopic dermatitis, and cerebral infarction, which comprises a peptide selected from the following:

(a) a peptide comprising the amino acid sequence of SEQ ID NO: 1;
(b) a peptide comprising an amino acid sequence resulting from substitution, deletion, insertion, or addition of one or several amino acids in the amino acid sequence of SEQ ID NO: 1; and
(c) a peptide comprising an amino acid sequence with a sequence identity of about 90% or higher with the amino acid sequence of SEQ ID NO: 1.

10. A peptide selected from the following:

(a) a peptide comprising the amino acid sequence of SEQ ID NO: 1;
(b) a peptide comprising an amino acid sequence resulting from substitution, deletion, insertion, or addition of one or several amino acids in the amino acid sequence of SEQ ID NO: 1; and
(c) a peptide comprising an amino acid sequence with a sequence identity of about 90% or higher with the amino acid sequence of SEQ ID NO: 1.

FIG. 1

FIG. 2

FIG. 3

FIG. 4

FIG. 5

Control                                    1r10

FIG. 6

FIG. 7

Control

1r10

FIG. 8

FIG. 9

Control

1r10

FIG. 10

FIG. 11

FIG. 12

## INTERNATIONAL SEARCH REPORT

| International application No. |
| --- |
| PCT/JP2019/039232 |

**A. CLASSIFICATION OF SUBJECT MATTER**
Int.Cl. A61K38/16(2006.01)i, A61P1/04(2006.01)i, A61P29/00(2006.01)i,
A61P37/02(2006.01)i, A61P43/00(2006.01)i

According to International Patent Classification (IPC) or to both national classification and IPC

**B. FIELDS SEARCHED**

Minimum documentation searched (classification system followed by classification symbols)
Int.Cl. A61K38/16, A61P1/04, A61P29/00, A61P37/02, A61P43/00

Documentation searched other than minimum documentation to the extent that such documents are included in the fields searched
Published examined utility model applications of Japan    1922-1996
Published unexamined utility model applications of Japan    1971-2019
Registered utility model specifications of Japan    1996-2019
Published registered utility model applications of Japan    1994-2019

Electronic data base consulted during the international search (name of data base and, where practicable, search terms used)
JSTPlus/JMEDPlus/JST7580(JDreamIII), CAplus/MEDLINE/EMBASE/BIOSIS(STN),
UniProt/GeneSeq

**C. DOCUMENTS CONSIDERED TO BE RELEVANT**

| Category* | Citation of document, with indication, where appropriate, of the relevant passages | Relevant to claim No. |
| --- | --- | --- |
| A | WO 2011/052668 A1 (GENOMIX CO., LTD.) 05 May 2011, claims, examples<br>& US 2012/0251510 A1, claims, examples & EP 2494977 A1 & JP 2016-34964 A & JP 2017-137350 A | 1-10 |
| A | WO 2009/133939 A1 (GENOMIX CO., LTD.) 05 November 2009, claims, examples<br>& US 2011/0091928 A1, claims, examples & EP 2301559 A1 | 1-10 |

☒ Further documents are listed in the continuation of Box C.    ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 21.10.2019 | 05.11.2019 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

33

**INTERNATIONAL SEARCH REPORT**

International application No.

PCT/JP2019/039232

C (Continuation). DOCUMENTS CONSIDERED TO BE RELEVANT

| Category* | Citation of document, with indication, where appropriate, of the relevant passages | Relevant to claim No. |
|---|---|---|
| A | 何 彦霆, 他 4 名, HMGB1 ameliorates inflammatory bowel disease by inducing circulating mesenchymal stem cells, 第 17 回日本再生医療学会総会 プログラム抄録 [online] retrieved on 21 October 2019, 23 February 2018, 0-34-2, retrieved from the Internet, URL: http://www2.convention.co.jp/17jsrm/appli/から取得, entire text, (HE, Y. T. and 4 others, The 17th Congress of the Japanese Society for Regenerative Medicine, Program and Abstracts) | 1-10 |
| A | YAMAOKA, S. et al., Systemic delivery of HMGB1 peptide ameliorates imiquimod-induced psoriasis-like dermatitis, Journal of Investigative Dermatology, May 2018, 138 (5 supplement), p. S177, 1043, entire text | 1-10 |
| P, A | WO 2018/186480 A1 (STEMRIM INC.) 11 October 2018, claims, examples (Family: none) | 1-10 |
| P, A | WO 2019/107530 A1 (STEMRIM INC.) 06 June 2019, claims, examples & WO 2019/107566 A1 | 1-10 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008053892 A
- WO 2009133939 A
- WO 2012147470 A

**Non-patent literature cited in the description**

- *PNAS,* 19 April 2011, vol. 108 (16), 6609-14
- *Gene,* 1988, vol. 67, 31-40
- *Bio/Technology,* 1988, vol. 6, 47-55
- **MARSHAK et al.** Strategies for Protein Purification and Characterization: A Laboratory Course Manual. Cold Spring Harbor Laboratory Press, 1996
- Remington's Pharmaceutical Science. Mark Publishing Company
- **LI et al.** *PLoS One.,* 07 December 2015, vol. 10 (12), e0144101
- **LI M et al.** *Proc Natl Acad Sci USA.,* 2006, vol. 103, 11736-41
- **PAXINOS G. ; WATSON C.** The rat brain in stereotaxic coordinates. Academic Press Inc, 1986